# EUROPEAN PATENT SPECIFICATION

(11) **EP 0 570 915 B1**
(45) Date of publication and mention of the grant of the patent: **29.10.1997**
(21) Application number: 93108098.0
(22) Date of filing: 18.05.1993
(51) Int. Cl.: A61B 19/00, A61B 17/115

(54) **Surgical apparatus for delivering the anvil of an anastomosis device**
Vorrichtung zum Abgeben eines Ambosses einer Anastomosevorrichtung
Appareil chirurgical pour délivrer une enclume d'un dispositif pour l'anastomose

(30) Priority: 19.05.1992 US 886040; 23.09.1992 US 950435
(43) Date of publication of application: 24.11.1993
(73) Proprietor: United States Surgical Corporation, Norwalk, Connecticut 06856 (US)
(72) Inventor: Green, David T., Westport, CT 06880 (US); Bolanos, Henry, E. Norwalk, CT 06855 (US); Sienkiewicz, Henry, Stamford, CT 06903 (US); Leahy, Patrick F., Dublin 4 (IE)
(74) Representative: Marsh, Roy David

(56) References cited:
- EP-A- 0 282 157
- WO-A-87/06448

## Description

### BACKGROUND OF THE INVENTION

### 1. Field of the Invention

This invention relates to a surgical delivery apparatus, and in particular, to an apparatus for delivering an anvil component to a remote location within a hollow organ to effect the joining of hollow organ sections by circular anastomosis.

Such apparatus is disclosed in WO87/06448 which is used as basis for the preamble of claim 1. A probe extends into an axial bore from the hemispherical rear face of an anvil having a flat front face transverse to the axial bore. An anvil shaft extends outwardly from the centre of the flat face, along the axis of the anvil. On the shaft, and covering the front face, is fitted a paraboloid cap, thereby to enable the anvil to be introduced, by pushing it with the probe into the interior of the body cavity which is the intended site of surgery. The cap by then has served its function and has to be removed.

### 2. Description of the Prior Art

Circular anastomosis is the surgical joining of separate hollow organ sections so that the sections intercommunicate. Typically, the anastomosis procedure follows surgery in which a diseased or defective section of hollow tissue is removed and the remaining end sections are to be joined. In accordance with such procedures, the operative tissue is exposed by making several extensive incisions in the body cavity wall and folding the cut tissue to provide access to the surgical site. The diseased section of the organ is removed thereby leaving two separate end sections of organ which are thereafter fastened by means of a stapling instrument which drives a circular array of staples through the end sections and simultaneously cores out any overlapping tissue to free the tubular passages.

### SUMMARY OF THE INVENTION

The present invention as defined in claim 1 provides a surgical apparatus for delivering an anvil component to a targeted section of a tubular organ so that anastomosis of two separated organ sections may be achieved using minimally invasive surgical techniques.

Generally stated, the anvil delivering apparatus includes an elongated delivery member, a mounting mechanism for detachably mounting the anvil component on a distal end of the elongated delivery member and releasing means for releasing the anvil component from the mounting means and into the targeted tubular organ section. The delivery member engages with the shaft of the anvil, so the flat face of the anvil faces proximally during insertion of the anvil.

In a preferred embodiment, the surgical apparatus includes an elongated delivery member having a proximal and a distal end, a rod member extending coaxially within the elongated delivery member and having a bearing surface at its distal end and a mounting portion for detachably mounting the anvil component at the distal end of the elongated delivery member. The rod member is adapted for distal displacement relative to the elongated delivery member such that the bearing surface of the rod member engages the anvil component and causes release of the anvil component from the mounting portion and expels the anvil component into the targeted tissue section.

In a preferred embodiment, the anvil delivery system includes an elongated delivery member having a proximal and a distal end and a longitudinal bore extending therethrough and a hand grip portion disposed at the proximal end of the elongated delivery member to facilitate handling of the delivery system. The hand grip portion includes a longitudinal bore extending therethrough in axial alignment with the bore of the elongated delivery member. The system further includes a rod member extending coaxially within the longitudinal bores of the elongated delivery member and the hand grip portion. The rod member is adapted for longitudinal movement towards the distal end of the elongated delivery member. Preferably a proximal end portion of the rod member extends beyond a proximal end of the hand grip member. An anvil component is mounted at the distal end of the elongated delivery member.

In use, the distal end of the elongated delivery member with mounted anvil component is inserted within the hollow organ and advanced to a desired location in the tubular organ. A distal force is applied to the proximal end portion of the rod member, which causes the rod member to distally advance and engage the anvil component to effect release of the anvil component from its engagement with the elongated delivery member to expel the anvil component within the desired organ section.

The present invention helps to make available a method for performing circular anastomosis of first and second intestinal sections using minimally invasive surgical techniques. The method comprises providing an anvil delivery system, including an elongated delivery member having a proximal and a distal end, mounting means for releasably mounting an anvil component to the distal end of the elongated delivery member and releasing means for releasing the anvil component from the mounting means to expel the anvil component within the targeted organ section. In accordance with the method, the distal end of the elongated delivery member with mounted anvil component is transanally inserted and advanced into the intestine until the anvil component is disposed beyond a diseased tissue section. The releasing means is thereafter actuated to release the anvil component from the mounting means and to place the anvil component within an intestinal section beyond the diseased Section. The anvil delivery system is then withdrawn from the operative site.

Thereafter, a first and second side of the diseased tissue section is isolated and the diseased section is resected, preferably by laparoscopic means, leaving first and second intestinal sections having first and second stapled ends, respectively, with the anvil component disposed within the second intestinal section. An opening is made in the second stapled end of the second intestinal section so that the anvil component may be grasped and exposed. An apparatus for performing circular anastomosis of the first and second intestinal sections is introduced transanally and advanced into the first intestinal section until a distal end of the apparatus engages the first stapled end. An opening is made in the first stapled end to expose the staple holding component of the apparatus. The anvil shaft is then mounted within the staple holding component. This mounting interposes the two ends of the intestinal sections between the anvil component and the staple holding component. The apparatus is fired and the anastomosis of the first and second intestinal sections is completed. Thereafter, the apparatus is removed from the body.

Generally stated, the present invention is embodied in an anvil assembly and associated delivery system, the anvil assembly being adapted for use with an apparatus for performing circular anastomosis. The anvil assembly comprises an elongated anvil rod having proximal and distal end portions and an anvil head which can be detachably mounted to the distal end portion of the anvil rod. The distal end portion can be pivotally mounted and adapted to pivot from a first operative position to a second non-operative position, whereby at least one dimension of the anvil assembly in the second non-operative position is effectively less than the corresponding dimension in the first operative position.

In a preferred embodiment, the anvil assembly comprises an elongated anvil rod having proximal and distal end portions and an anvil head detachably mounted to the distal end portion of the anvil rod. The distal end portion is pivotal from a first operative position in general alignment with a longitudinal axis defined by the anvil rod to a second non-operative position angularly displaced relative to the longitudinal axis. In the second non-operative position, the anvil rod presents a less obtrusive profile which, accordingly, facilitates advancement of the anvil assembly through body tissue.

The distal end portion is pivotal with respect to the longitudinal axis defined by the anvil rod through an angle of up to about 90°. In particular, the distal end portion is adapted to pivot up to about 90° with respect to each side of the longitudinal axis, thereby providing full pivotal articulation thereof of about 180°.

The distal end portion comprises a circumferential mounting collar which is received within a circular aperture formed within the anvil head to mount the anvil head to the anvil rod. The mounting collar preferably comprises a plurality of longitudinally extending external splines which are engagable with cooperating longitudinally extending internal splines formed within the anvil head to properly align the anvil head with the anvil rod.

The anvil rod also comprises a plurality of longitudinally extending external splines disposed intermediate its proximal and distal end portions. The external splines are engagable with cooperating longitudinally extending internal splines formed within a distal end of the apparatus to properly align the anvil rod with the apparatus.

The anvil apparatus is adapted to be mounted to an elongated delivery member which includes a mounting mechanism for detachably mounting the anvil assembly on a distal end of the elongated delivery member and releasing means for releasing the anvil member from the mounting means.

The surgical apparatus for performing circular anastomosis of first and second tissue sections comprises elongated tubular means having a proximal and a distal end, means for firing a plurality of fasteners from the distal end of the elongated tubular means and anvil means detachably mounted to the distal end of the elongated tubular means. The anvil means comprises an anvil rod having proximal and distal end portions and an anvil head which can be detachably mounted to the distal end portion of the anvil rod. The distal end portion can be made pivotal from a first operative position in general alignment with a longitudinal axis defined by the anvil rod to a second non-operative position angularly displaced relative to the longitudinal axis. The anvil assembly defines an effective cross-sectional area generally transverse to the longitudinal axis. The effective cross-sectional area of the anvil assembly in the second non-operative position is less than the effective cross-sectional area of the anvil assembly in the first operative position to facilitate introduction and advancement of the anvil assembly through body tissue.

### BRIEF DESCRIPTION OF THE DRAWINGS

FIG. 1 is a perspective view of the anvil delivery system of the present invention;
FIG. 2 is a side view of the delivery system of FIG. 1 with a partial cutaway of the distal-end, illustrating the positioning of the anvil shaft of the anvil component and tail portion within the elongated sheath member when the apparatus is in the pre-fired condition;
FIG. 3 is a perspective view of the delivery system of FIG. 1 in the post-fired condition with the anvil component expelled from the elongated sheath member;
FIG. 4 is a perspective view of the delivery system of FIG. 1 inserted transanally and extending through a portion of the intestine;
FIG. 5 is a perspective view of the delivery system of FIG. 1 inserted through the intestine and in the post-fired condition with the anvil component and tail portion expelled from the delivery system;
FIG. 6 is a perspective view illustrating removal of a diseased tissue section by a laparoscopic stapling instrument;
FIG. 7 is a perspective view after application of the laparoscopic stapling instrument, illustrating the formed first and second intestinal sections;
FIG. 8 is a perspective view with a partial cut-aways of the first intestinal section, illustrating advancement of an apparatus for performing anastomosis and exposure of the anvil shaft from the second intestinal section;
FIG. 9 is a perspective view with partial cutaways of the first and second intestinal sections, illustrating the anvil shaft of the anvil component mounted within the staple holding component;
FIG. 10 is a perspective view of the anastomosis of the first and second intestinal sections after firing of the anastomosis apparatus;
FIG. 11 is a perspective view illustrating an alternative method for resection and removal of the diseased tissue section in which the diseased section is to be removed through the rectal opening;
FIG. 12 is a perspective view after removal of the diseased section through the rectal opening and application of a laparoscopic stapling instrument to the first intestinal section;
FIG. 13 is a perspective view of a surgical stapler apparatus for performing anastomosis of hollow organs;
FIG. 14 is a side plan view of an alternative embodiment of the detachable anvil rod illustrating the pivotal distal end portion;
FIG. 15 is a sectional view with parts separated of the distal end portion and the remaining portion of the rod of FIG. 14 illustrating the mounting components for pivotally mounting the distal end portion;
FIG. 16 is a side plan view of the detachable anvil rod of FIG. 14 with mounted anvil head illustrating the distal end portion of the rod in a generally aligned operative position;
FIGS. 17A-17B are side plan views similar to the view of FIG. 16 illustrating the distal end portion of the anvil rod pivoted 90° with respect to each side of the longitudinal axis defined by the anvil rod;
FIG. 18 is a side view of a delivery system used to deliver the anvil rod to a desired predetermined location within a tubular organ;
FIG. 19 is a perspective view of the intestinal area of a patient illustrating the introduction of the surgical apparatus of FIG. 13 prior to mounting of the anvil rod of the present invention to the apparatus; and
FIG. 20 is a perspective view similar to that of FIG. 19 illustrating mounting of the anvil rod of the present invention to the distal end of the surgical apparatus.

### DESCRIPTION OF THE PREFERRED EMBODIMENTS

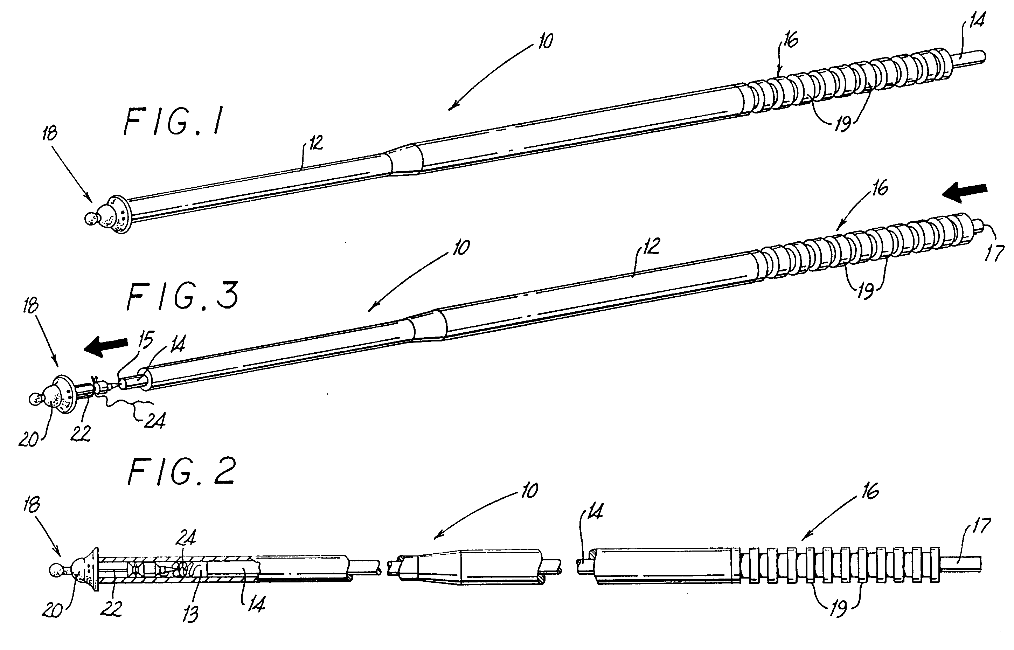

Referring now to the drawings and, in particular, to FIGS. 1-3, there is shown an anvil delivery system in accordance with one embodiment of the present invention. Anvil delivery system 10 includes an elongated sheath member 12 having longitudinal bore 13 (see FIG. 2) extending therethrough, rod member 14, and hand grip member 16 disposed at the proximal end portion of sheath member 12. Hand grip member 16 also includes a longitudinal bore extending therethrough in axial alignment with longitudinal bore 13 of sheath member 12. In a preferred embodiment, hand grip member 16 includes a grip enhancing means such as a plurality of circumferential ribs 19 to facilitate grasping and maneuvering of the delivery system.

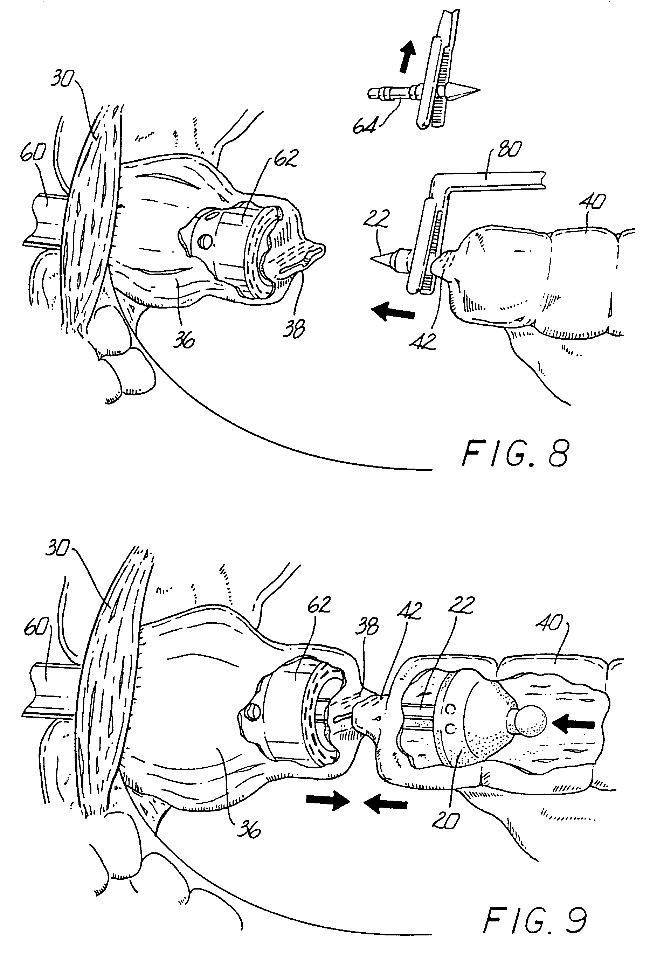

Anvil delivery system 10 expels anvil component 18 into a hollow tubular tissue section which is to be subsequently attached to an adjacent tissue section by circular anastomosis. Anvil component 18 includes anvil head 20 mounted on anvil shaft 22. Anvil shaft 22 is intended to be mounted within staple holding component 62 (see FIG. 8) of a stapling apparatus. Anvil head 20 may include an annular array of staple forming buckets to receive and bend staples fired from staple holding component 62. It is to be noted that the components of delivery system 10 can be modified to accommodate a variety of sizes and types of anvils.

Rod member 14 is slidably received within longitudinal bore 13 of sheath member 12 and the longitudinal bore of hand grip member 16, and is adapted to move in a distal direction relative to the remaining components in the delivery system. In a preferred embodiment, proximal end portion 17 of rod member 14 extends beyond the proximal end of hand grip member 16 as best shown in FIGS. 1 and 2. When a force is applied to proximal end portion 17, rod member 14 slides in a distal direction as shown by the arrow in FIG. 3. This distal movement causes bearing surface 15 of rod member 14 to engage shaft 22 of anvil component 18 and to release anvil component 18 from its engagement with elongated sheath member 12, and to expel the component into a targeted tissue section.

Anvil component 18 may be mounted to elongated sheath member 12 by conventional means. In the embodiment and as best shown in FIG. 2, anvil shaft 22 is inserted within the distal end of sheath member 12 to mount the anvil component. Preferably, the diameter of shaft 22 is slightly less than the inner diameter of the proximal end of sheath member 12 such that the peripheral surface of shaft 22 frictionally engages the inner peripheral surface of sheath member 12 to assist in retaining anvil component 18 within the sheath member during insertion of the system within the tubular organ.

Delivery system 10 may range in length from about 60 cms to about 180 cms, however, any appropriate length may be chosen depending upon the particular application. In a preferred embodiment system 10 is straight, however, it is within the scope of the present invention for system 10 to be curved to facilitate placement of the instrument in particular body structures and to reach remote or relatively inaccessible operative sites. It is also possible for delivery system 10 to be flexible. Preferably all components of delivery system 10 are fabricated from polymeric materials, which thereby reduces the cost of manufacture of the system and makes it economically feasible to dispose the system after use.

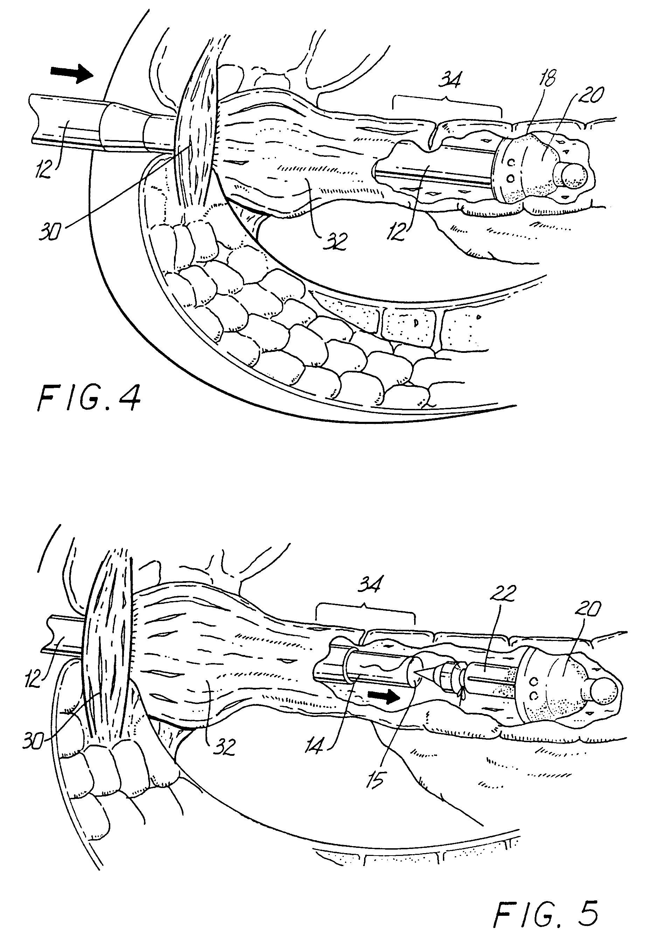

Further understanding of the significant aspects of the delivery system of the present invention will become more readily apparent by the following description of the use of same. Referring now to FIGS. 4 - 10, the anvil delivery system 10 in accordance with the present invention is shown in a sequence of operation.

Referring initially to FIG. 4, the surgeon grasps delivery system 10 by hand grip 16 and inserts the distal end of the system with mounted anvil component 18 through the rectal opening 30 and into colon or intestine 32. The system 10 is then advanced within intestine 32 until anvil head 20 extends slightly beyond diseased section 34 of the intestine.

Referring now to FIG. 5, the delivery system is actuated by the surgeon by depressing proximal end portion 17 of rod member 14 (see FIGS. 1-3) to thereby effect distal movement of the rod member and cause bearing surface 15 of the rod member to engage the proximal end of shaft 22 and eject anvil component 18 into intestine 32. It is to be appreciated that after release from system 10, anvil component 18 is disposed in a section of intestine beyond diseased section 34. At this point in the procedure, delivery system 10 is removed from the colon.

The diseased tissue section is then excised followed by anastomosis of the adjacent severed tissue sections. Preferably, the remaining operative procedures will be performed using minimally invasive surgical techniques including laparoscopic means and instrumentation. This is a significant aspect of the present invention and is made possible by the prior transanal placement of the anvil component within the intestinal tissue. Such placement removes the need for incising the abdominal cavity to introduce the detached component to the operative site.

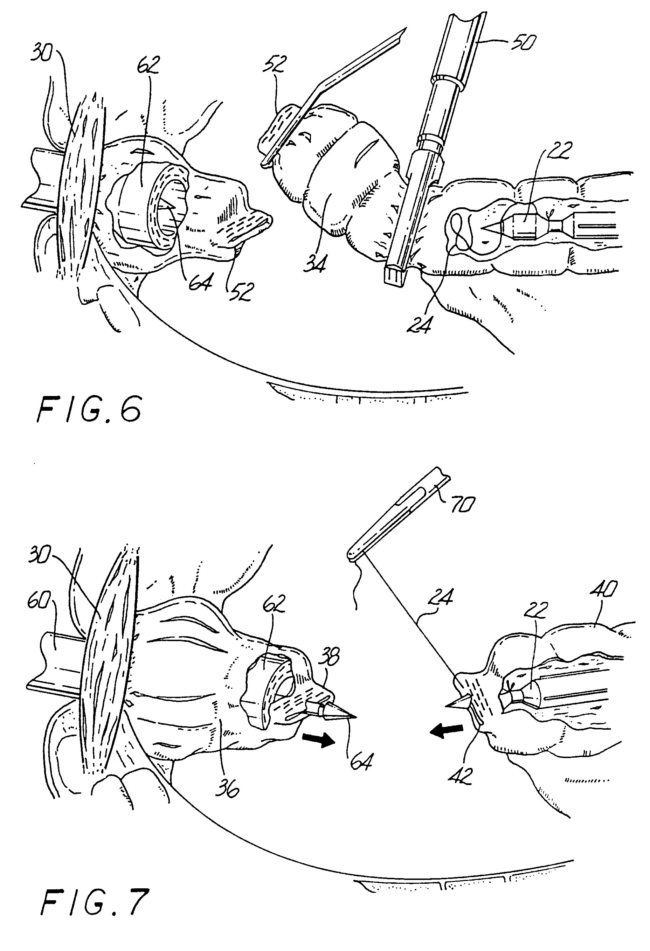

Referring now to FIG. 6, the preferred method for resecting diseased section 34 is illustrated. Through appropriate trocar sleeves, the surgeon applies a laparoscopic stapler 50 to both sides of diseased section 34. A suitable stapler for this purpose is described in commonly assigned U.S. Patent No. 5,040,715 issued August 20, 1991. Each application of the stapler places two triple staggered rows of staples 52 while a knife cuts therebetween. FIG. 6 illustrates the staple rows after the application of stapler 50 to a section of the intestine nearest rectal opening 30. FIG. 6 also shows stapler 50 being applied to a section of the intestine beyond diseased section 34.

Referring now to FIG. 7, after application to both sides of diseased section 34, stapler 50 will have created two separated, closed end tissue sections 36, 40 having stapled ends 38, 42, respectively. This application will also have severed diseased section 34 from the remaining intestinal tissue. In FIG. 7, diseased section 34 is already removed from the operative site, preferably through one of the trocar sleeves (not shown). At this point in the procedure, anvil component 18 is positioned within tissue section 40.

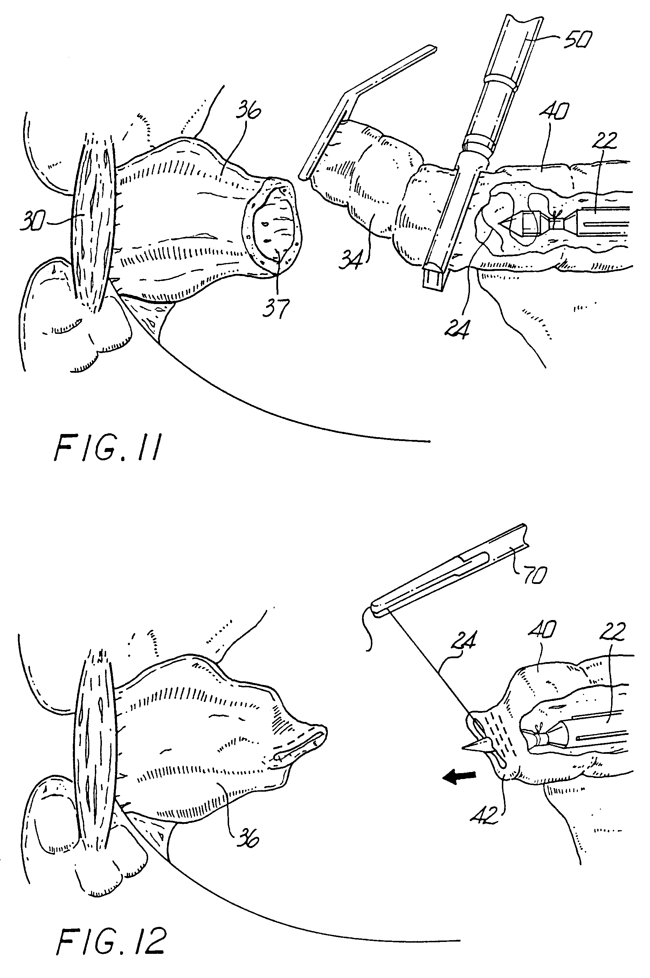

Alternative laparoscopic instruments and methods may be incorporated to isolate diseased section 34 from the remaining intestinal tissue and to remove section 34 from the operative site. Referring to FIG. 11, the lower side of diseased section 34 may be severed from the intestinal tissue by, for example, a conventional scalpel. This step in the procedure creates intestinal section 36 having open end 37. Thereafter, stapling instrument 50 may be applied to the upper side of section 34 to close off and completely sever the diseased section from the intestine. Since the end of tissue section 36 is not closed, open end 37 provides an avenue to remove diseased section 34, i.e., removing the diseased section through the rectal opening 30. After removal, intestinal section 36 may be closed off as shown in FIG. 12 by conventional means, such as, for example, with a laparoscopic stapling instrument. Alternatively, tissue section 36 may be closed off around the staple holding component by a purse string suture.

The upper side of diseased section 34 may be closed off with a laparoscopic stapling instrument which fires a single row of staples, and then sever the diseased section on its upper side with a scalpel, laser or electrocautery device which is applied through a trocar sleeve. Thereafter, a similar cutting device may be used on the lower side of section 34 and the completely severed diseased section removed through end section 36 and out rectal opening 30 in the same manner as previously described. Intestinal section 36 may then be closed off with the stapling instrument. It is also possible to isolate diseased section 34 using a scalpel on both sides of the diseased section and to leave the ends of intestinal sections 36,40 open. Sections 36,40 may subsequently positioned for attachment by conventional drawstring or purse string sutures.

Referring to Figs. 7-10 rejoining of the tissue ends is accomplished by inserting an apparatus 60 for performing circular surgical stapling of hollow tissue organs through rectal opening 30 and into intestinal section 36. Apparatus 60 includes staple holding component 62 with trocar 64 detachably mounted therewithin. Apparatus 60 may be any known instrument that is adapted to be inserted transanally to perform circular anastomosis of tissue sections. Examples of such instruments are described in commonly assigned U.S. Patent Nos. 4,304,236, 4,379,457, 4,573,468, 4,576,167, 4,603,693 and 4,646,745. Apparatus 60 is advanced into section 36 such that staple holding component 62 approaches stapled end 38 and trocar 64 contacts and penetrates the stapled end to form an incision through end 38 to expose the staple holding component. After the incision is formed in stapled end 38, the surgeon, through an appropriate trocar sleeve creates an opening with forceps or the like in stapled end 42 of intestinal section 40 and probes within the opening to locate and grasp anvil shaft 22. Preferably, the anvil assembly is provided with a tail 24 to assist in locating and removing the anvil assembly. Tail 24 is a section of thread or the like and is secured to shaft 22 prior to insertion of the delivery system to facilitate withdrawal of the shaft through the opening in stapled end 42. As shown, tail 24 is removed from the opening and pulled away from end 42 by forceps 70 until anvil shaft 22 becomes visible.

Referring to FIG. 8, trocar 64 is released from its engagement with staple holding component 62 and removed from the operative site through one of the trocar sleeves. Thereafter, the surgeon grasps anvil shaft 22 with grasping tool 80 and pulls the shaft until a portion of the shaft is exposed. At this point in the procedure, intestinal sections 36 and 40 are ready to be joined together by circular anastomosis. Intestinal sections 36, 40 are properly drawn over and secured around staple holding component 62 and anvil component 18, respectively, without maneuvering the tissue around these components. The intestinal sections 36, 40 are secured in position for attachment without requiring the use of conventional drawstrings or purse string techniques to tighten the tissue sections around their respective stapler components.

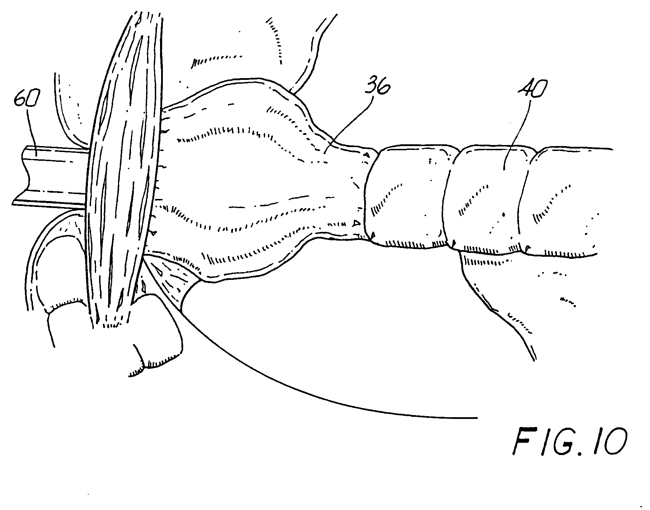

Referring now to FIG. 9, through appropriate trocar sleeves, intestinal sections 36,40 are approximated and anvil shaft 22 is mounted within staple component 62. This mounting properly interposes stapled ends 38, 42 of the intestinal sections 36, 40 between staple holding component 62 and anvil head 20, respectively. Thereafter, the stapling instrument is fired to perform the anastomosis. The excess portion of the ends of tissue portions are severed by the action of a knife edge incorporated in the anastomosis instrument. FIG. 10 illustrates the anastomosis of intestinal sections 36, 40 after firing of the surgical apparatus. The surgeon thereafter removes the instrument with attached anvil component from the surgical site.

The present invention provides a novel device for delivery of an anvil component within a tubular organ which avoids the use of conventional surgery to expose the targeted tissue portion. The device can be manufactured cost-effectively and disposed of after use. The device makes it possible to perform anastomosis of hollow tissue sections entirely by laparoscopic techniques.

Although the present invention has been shown and described in terms of a preferred embodiment, it will be appreciated that various changes and other modifications are contemplated within the scope of the present invention as defined by the following claims.

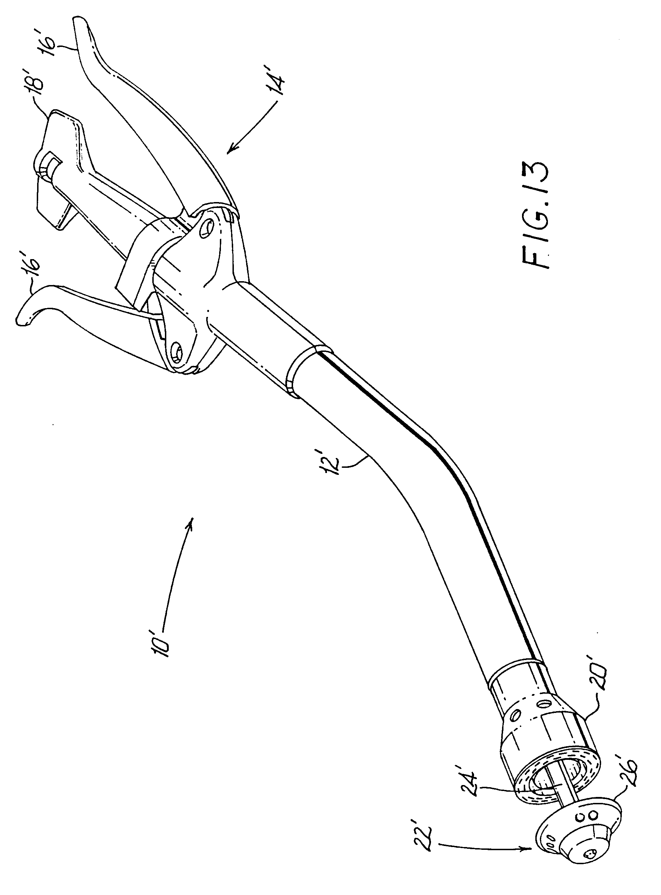

Referring to FIG. 13, there is illustrated an apparatus 10' for stapling hollow tubular body organs as by circular anastomosis of intestines, colons, or the like. The apparatus may be utilized to attach two tubular body parts or one tubular body part to a non-tubular body part by circular anastomosis and may be adapted to attach the body parts with deformable metallic staples or bio-absorbable two-part body tissue fasteners.

Such apparatus 10' is disclosed and claimed in U.S. Patent No. 5,119,983, issued June 9, 1992. This apparatus is a stapler for anastomosis of hollow body organs such as intestines, colons, etc. Other such devices are disclosed in the following U.S patents: U.S. Patent No. 4,304,236, issued December 8, 1981; U.S. Patent No. 4,379,457, issued April 12, 1983; U.S. Patent No. 4,573,468, issued March 4, 1986; U.S. Patent No. 4,576,167, issued March 18, 1986; U.S. Patent No. 4,603,693, issued August 5, 1986; U.S. Patent No. 4,646,745, issued March 3, 1987 and U.S. Patent No. 5,122,156, issued June 16, 1992. As will be appreciated from a review of these patents, such devices in some instances may also be manually operated and are sometimes controlled from a location remote from the point of manipulation.

Generally, apparatus 10' includes elongated shaft 12' and handle mechanism 14' attached to a proximal end of the elongated shaft. Handle mechanism 14' includes actuating handles 16' and adjusting wing nut 18'. Fastener retainer component 20' is connected to the distal end of shaft 12' and houses an annular array of staples therein. A staple firing mechanism expels the staples from fastener retainer component 20'.

An anvil assembly 22' is detachably mounted to the distal end of elongated shaft 12' by a mounting mechanism within the shaft which cooperatively engages the anvil assembly. Anvil 22' includes detachable anvil rod 24' with attached anvil head 26'. Anvil head 26' includes staple receiving buckets (not shown) for receiving and clinching the staples expelled by the staple firing mechanism to thereby join the adjacent tissue sections.

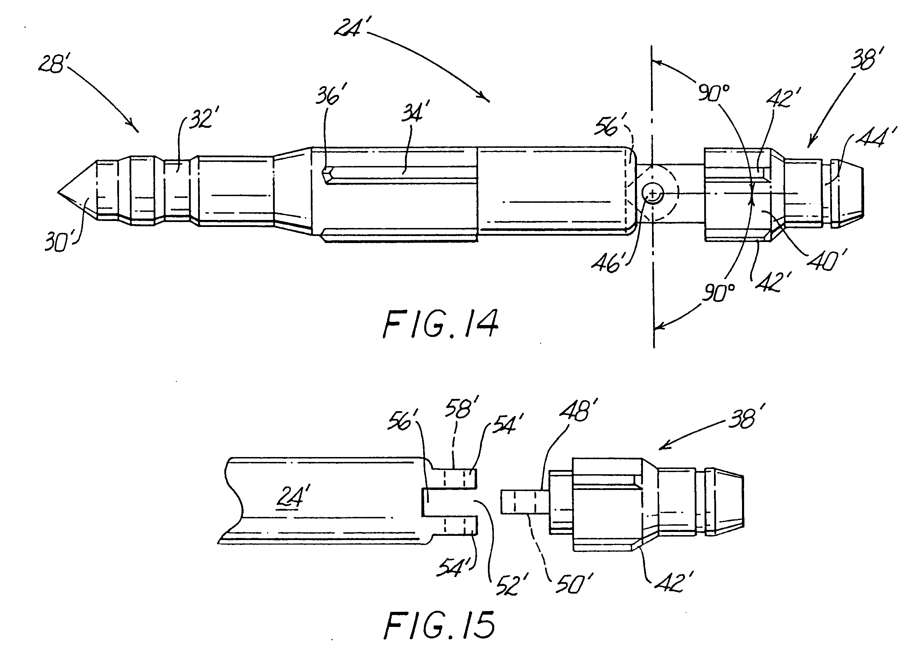

Referring now to FIG. 14, there is illustrated the detachable anvil rod 24'. Anvil rod 24' is adapted to be used with apparatus 10' and includes proximal end portion 28' which is received within and engages the mounting mechanism within the distal end of elongated shaft 12'. (FIG. 13) Proximal end portion 28' includes a generally conical shaped mounting portion 30' which is advantageously dimensioned to facilitate entry within the distal end of apparatus 10' and which further enables manipulation of rod 24' through body tissue. Proximal end portion 28' also includes an annular recess 32' which is correspondingly configured to be engaged by the mounting mechanism within the distal end of elongated shaft 12' of the apparatus.

A plurality of longitudinally extending external splines 34' are disposed in the general midportion of anvil rod 24'. Splines 34' engage correspondingly configured and positioned longitudinal internal splines in the distal end of elongated shaft 12' during mounting of the rod to the apparatus to ensure proper alignment of the rod with the staple firing mechanism. Each external spline 34' has a chamfered and sloped proximal end 36'. Chamfered ends 36' engage the internal splines within elongated shaft 12' and cause the rod to rotate slightly if the internal and external splines are initially misaligned during mounting so as to ensure proper mating between the two components.

Distal end portion 38' of anvil rod 24' includes an anvil head mounting collar 40' for mounting anvil head 26' to the rod. Collar 40' includes a plurality of longitudinally extending external splines 42' which engage with cooperating longitudinally extending internal splines in the anvil head 26' to properly align the staple-receiving buckets in the anvil head with the staples in fastener retainer component 20'. A circumferential groove 44' is formed adjacent collar 40' and is adapted to receive a U-shaped clamp which securely retains the anvil head on the collar.

Distal end portion 38' of anvil rod 24' is pivotally mounted about pivoting pin 46' and is adapted to pivot from a position in general alignment with the remainder of rod 24' through pivoted locations (shown by the arrows) corresponding to plus or minus 90 degrees. As best shown in FIG. 15, distal end portion 38' includes a projecting member 48' having a generally circular aperture 50' (shown in phantom), which member 48' is received within a recess 52' defined between two correspondingly dimensioned and positioned projection members 54' extending from the main portion of rod 24'. Preferably, a slight groove 56' is formed in the main portion of rod adjacent recess 52' to accommodate projecting member 48' during pivoting action of distal end portion 38'. Projections 54' also include apertures 58' (shown in phantom) which align with aperture 50' formed in projecting member 48' of distal end portion 38' to receive pivoting pin 46' to effect the mounting. Other alternative methods for mounting distal end portion 38' to the main portion of rod 24' may be readily determined by one skilled in the art.

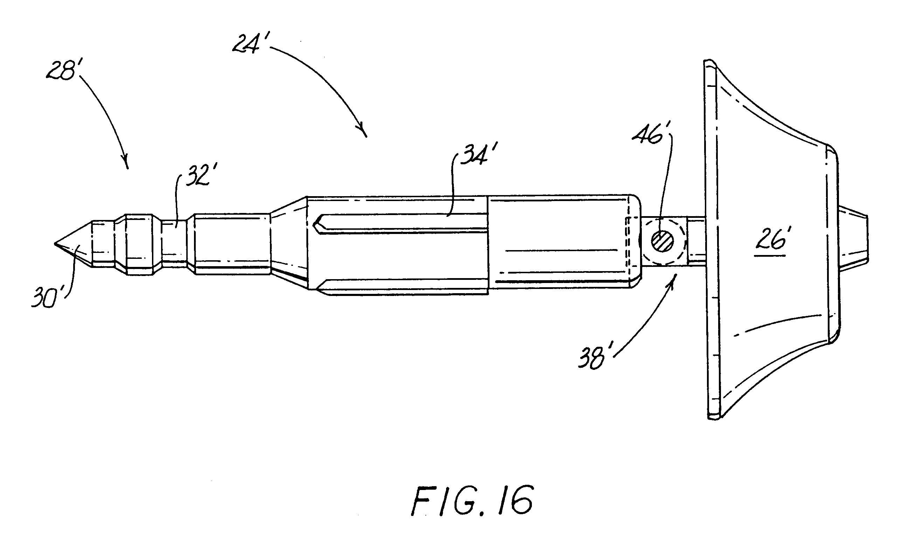

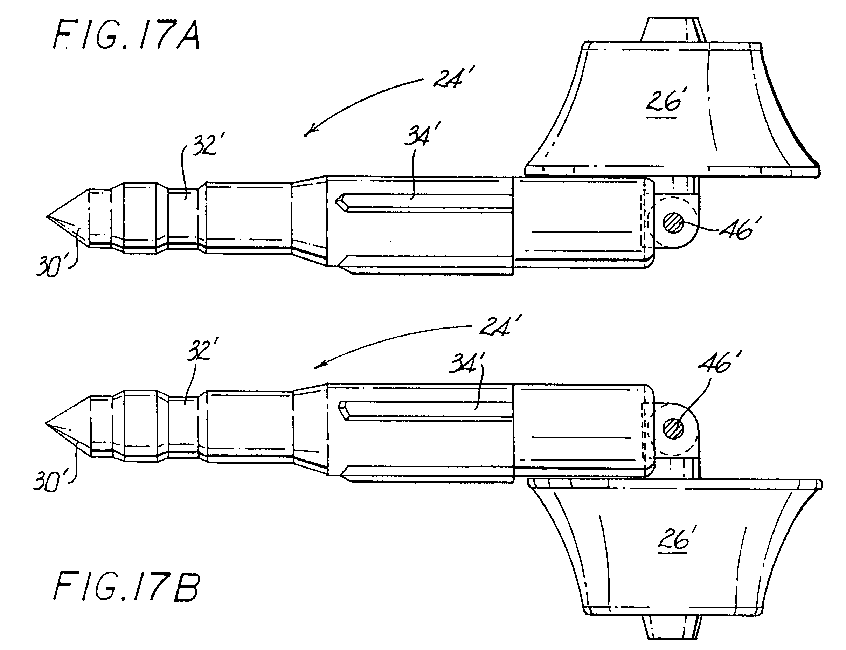

Referring now to FIGS. 16, 17A and 17B, anvil rod 24' is illustrated with anvil head 26' mounted on collar 40' so as to illustrate the advantages of the novel pivoting feature of distal end portion 38'. The pivoting feature of distal end portion 38' facilitates introduction and manipulation of anvil assembly 22' within tubular organ tissue such as the colon, intestines, etc. by orienting anvil head 26' in a manner which reduces the profile of the anvil head and the anvil assembly as the assembly is being advanced through the tubular organ. In particular, when distal end portion 38' is in a generally aligned position as shown in FIG. 16, which position corresponds to the orientation of a conventional anvil assembly, the anvil assembly 22' presents a relatively large cross-sectional dimension or profile which must pass through the tubular organ. Specifically, the dimension or profile is equal to the diameter of the anvil head, which in many instances, is greater than the corresponding inner dimension of the tubular organ in which it must pass. Consequently, anvil head 26' inherently engages the inner wall of the tubular organ during manipulation and advancement of the anvil assembly 22' and, accordingly, impedes such advancement within the tubular organ.

Referring now to FIGS. 17A and 17B, when distal end portion 38' is pivoted to its transverse position by way of the pivoting feature of the present invention, the effective transverse cross-sectional dimension or profile of anvil assembly 22' is substantially reduced. In particular, since anvil head 26' is generally parallel to and flush with anvil rod 24' in this position, the effective transverse cross-sectional dimension of assembly 22' is nearly one-half of the corresponding dimension in the operative position of anvil assembly shown in FIG. 16. It is to be appreciated that distal end portion 38' may be pivoted through a variety of angles relative to the anvil rod and still present a cross-sectional dimension or profile which is less than that of the generally aligned position of the distal end portion shown in FIG. 16.

In use, distal end portion 38' may be pivoted prior to introduction of anvil assembly within the tubular organ or may be initially inserted in a generally aligned position in which it subsequently assumes a pivoted position during advancement through the tubular organ due to engagement of anvil head 26' with the inner wall of the tubular organ.

In performing intestinal surgery such as a colonoscopy or a colectomy in which the surgery is followed by anastomosis of hollow tubular organs, anvil assembly 22' may be introduced into the hollow organ through a surgically provided incision, or transanally, and advanced to a predetermined location in the intestinal section so that the anvil assembly may be subsequently mounted to a stapling apparatus to complete the anastomosis.

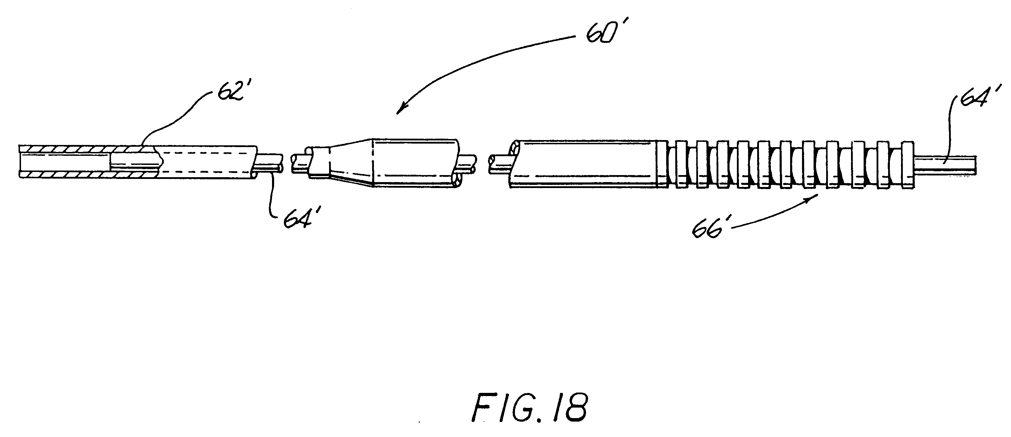

Referring now to FIG. 18, there is illustrated an apparatus which may be used to deliver anvil assembly 22' of the present invention to a predetermined desired location within a tubular organ section. System 60' is particularly adapted to deliver an anvil assembly transanally to a desired portion in the colon and includes an elongated sheath member 62' having a longitudinal bore, a pusher rod 64' slidably movable within the bore of the sheath member and hand grip member 66'.

In use with surgical apparatus of the present invention, anvil rod 24' is inserted within the distal end of sheath 62' to mount the anvil assembly 22' to the system. Thereafter, distal end portion 38' is pivoted to the position shown in either FIGS. 17A or 17B to reduce the effective transverse cross-sectional dimension of the anvil assembly 22'. The delivery system is inserted transanally and advanced through the colon to a predetermined desired location in the organ, preferably beyond the diseased section of tissue. The particular orientation of anvil head 24' facilitates introduction and advancement of anvil assembly 22' within the intestinal section. Thereafter, the delivery system is actuated by depressing the proximal end section 64a' of pusher rod 64', which extends beyond the proximal end of sheath 62', to advance the pusher rod such that it engages anvil rod 24' and expels the anvil assembly 22' from delivery system 60' and beyond the diseased tissue section. Once anvil assembly 22' is within the organ, the surgeon may perform the desired surgery followed by anastomosis of the hollow organ section.

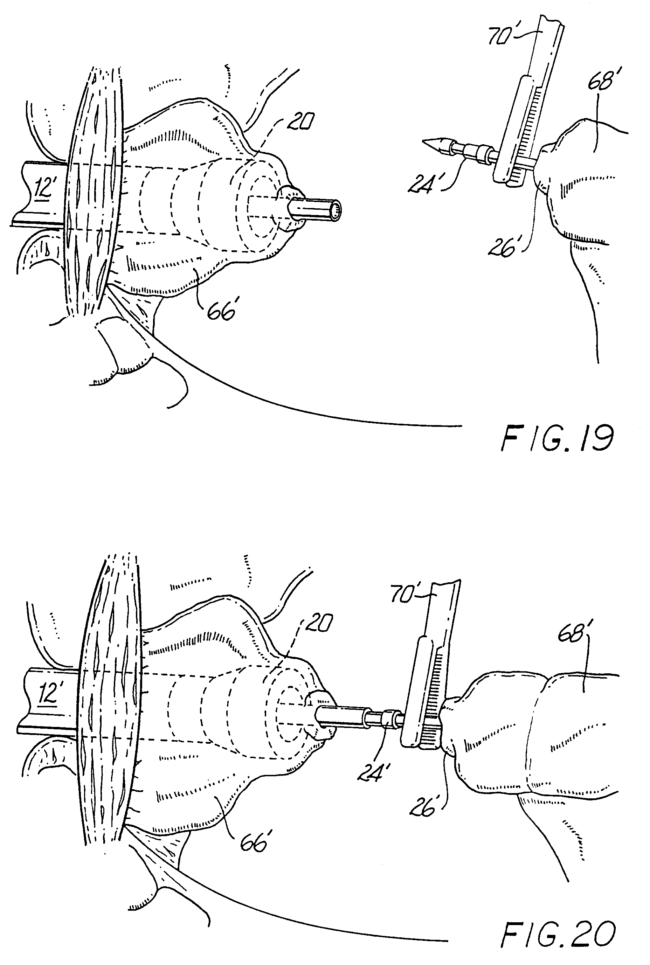

FIGS. 19 and 20 illustrate the use of apparatus 10' and detachable anvil rod 24' in an anastomosis procedure to effect joining of intestinal sections 66', 68'. Preferably, the anastomosis procedure is performed using minimally invasive surgical techniques including laparoscopic means and instrumentation. At the point in the procedure shown in FIG. 19, a diseased intestinal section had been previously removed preferably with a laparoscopic instrument applied to the operative site through an appropriate trocar sleeve. Elongated shaft 12' of apparatus 10' had been inserted transanally into intestinal section 66'. Both intestinal sections 66', 68' are also shown temporarily secured about their respective components by conventional means such as a purse string stitch.

In completing the anastomosis, the surgeon through an appropriate trocar sleeve probes within the intestinal section to grasp anvil rod 24', preferably with a grasping instrument 70' inserted within a cannula, and maneuvers rod 24' towards the distal end of elongated shaft 12'. Mounting portion 30' of rod 24' is then inserted within the distal end of elongated shaft 12' of the apparatus, as shown in FIG. 20, wherein the mounting mechanism within the distal end of the shaft engages the rod to effect the mounting. Thereafter, the anvil assembly and elongated shaft are approximated to clamp the opposed end portions of tissue between anvil head 26' and fastener retainer component 20'. Such approximation will also appropriately orientate the anvil head 26' with the apparatus. The apparatus is fired to complete the anastomosis.

## Claims

1. Surgical apparatus (10) for delivering an anvil to a site of surgery within a tubular organ section, which comprises:
an anvil (18) with an annular anvil head (20) and an axial anvil shaft (22) which extends along an axis from a transverse staple-forming face of the anvil head (20);
an elongate delivery member;
mounting means for releasably mounting the anvil at a distal end of said elongate delivery member; and
releasing means (14) for releasing the anvil from said mounting means.
**and characterised in that**
the mounting means is arranged to deliver the anvil in a distal movement, with the staple-forming face of the anvil directed proximally; and
the mounting means includes a sheath (12) at the distal end of said delivery member, the sheath having an open distal end which receives within it the shaft of the anvil.

2. Apparatus as claimed in claim 1, wherein said elongate delivery member includes a longitudinal bore extending therethrough, from its proximal end to its distal end.

3. Apparatus as claimed in claim 2 and including a hand grip portion (16) disposed at said proximal end of said elongate delivery member to facilitate handling of the delivery system, a longitudinal bore extending through said hand grip portion in axial alignment with said longitudinal bore of said elongate delivery member; and further including a rod member slidably received within said longitudinal bore of said elongate delivery member and said hand grip portion and adapted to move towards said distal end of said elongate delivery member, said rod member having a proximal end portion extending beyond a proximal end of said hand grip portion; and
wherein a distal force applied to said proximal end portion of said rod member causes said rod member to be distally displaced such that a distal end of said rod member engages said anvil shaft to effect release of said anvil component from its engagement with said distal end of said elongate delivery member.

4. Apparatus as claimed in claim 3, wherein an outer peripheral surface of said anvil shaft frictionally engages and inner peripheral wall of said elongate delivery member to assist in retaining said anvil component within said elongate delivery member.

5. Apparatus as claimed in any one of the preceding claims wherein a tail is secured to said anvil shaft.

6. Apparatus as claimed in any one of the preceding claims, and wherein:
(a) the anvil is an anvil assembly adapted for use with an apparatus for performing circular anastomosis, said anvil shaft comprising an anvil rod having proximal and distal end portions, said anvil head being detachably mounted to said distal end portion of said anvil rod, said distal end portion being pivotal from a first operative position to a second non-operative position, whereby at least one dimension of said anvil assembly in said second non-operative position is effectively less than the corresponding dimension in said first operative position.

7. Apparatus according to any one of the preceding claims, wherein said anvil shaft comprises a plurality of longitudinally extending external splines (34') disposed immediate its proximal and distal end portions, said external splines engageable with co-operating longitudinally extending internal splines formed within said sheath to properly align said anvil rod with the apparatus.

8. Apparatus according to any one of the preceding claims wherein the distal end of the anvil shaft comprises a circumferential mounting collar (40'), said mounting collar being received within a circular aperture formed within said anvil head to mount said anvil head to said anvil shaft.

9. Apparatus according to claim 8, wherein said mounting collar comprises a plurality of longitudinally extending external splines (42') engageable with co-operating longitudinally extending internal splines formed within said anvil head to properly align said anvil head with said anvil shaft.

10. Apparatus as claimed in any one of the preceding claims wherein the anvil head is detachably mounted to the distal end portion of said anvil shaft, said distal end portion being pivotal from a first operative position in general alignment with a longitudinal axis defined by said anvil shaft proximal of its distal portion to a second non-operative position angularly displaced relative to said longitudinal axis, whereby the assembly of anvil head and shaft defines an effective cross-sectional area generally transverse to said longitudinal axis and whereby the effective cross-sectional area of said anvil assembly in said second non-operative position is less than the effective cross-sectional area of said anvil assembly in said first operative position to facilitate advancement of said anvil assembly through body tissue.

11. Apparatus according to claim 10, wherein said distal end portion is pivotal with respect to said longitudinal axis defined by said anvil shaft through an angle of up to about 90°.

12. Apparatus according to claim 11, wherein said distal end portion is adapted to pivot up to about 90° with respect to each side of said longitudinal axis, whereby full pivotal articulation thereof is provided of about 180°.

13. Apparatus according to any one of the preceding claims wherein the proximal end portion of the anvil shaft includes a conical shaped mounting portion (30') configures and dimensioned to facilitate mounting of said anvil shaft to a receiving tube.

14. Apparatus as claimed in any one of the preceding claims, wherein said releasing means comprises a rod member received within said longitudinal bore of said elongate delivery member and having a bearing surface (15) at its distal end, said rod member adapted for distal displacement relative to said elongate delivery member such that said bearing surface engages the anvil component to effect distal displacement thereof and release of the anvil component from said mounting means.

15. Apparatus as claimed in any one of the preceding claims, wherein said mounting means comprises a frictional fit between an outer peripheral surface of an anvil shaft and an inner peripheral wall of said elongate delivery member.

16. Apparatus as claimed in any one of the preceding claims, wherein said elongate delivery member is flexible.

17. An anastomosis device (60) including apparatus as claimed in any one of the preceding claims including means for firing fasteners which are two-part absorbable fasteners.

## Patentansprüche

1. Chirurgische Vorrichtung (10) zum Abgeben eines Anschlags zu einem Operationsort innerhalb eines röhrenförmigen Organabschnittes, umfassend:
einen Anschlag (18) mit einem ringförmigen Anschlagkopf (20) und einem axialen Anschlagschaft (22) der sich entlang einer Achse von einer querverlaufenden klammerverformenden Fläche des Anschlagkopfs (20) erstreckt;
ein langgestrecktes Abgabeelement;
eine Befestigungseinrichtung, um lösbar den Anschlag an einem distalen Ende des langgestreckten Abgabeelementes anzubringen, und
eine Löseeinrichtung (14) um den Anschlag von der Befestigungseinrichtung zu lösen;
**dadurch gekennzeichnet, daß**
die Befestigungseinrichtung angeordnet ist, um den Anschlag in einer distalen Bewegung mit der klammerverformenden Fläche des Anschlags in proximaler Richtung gerichtet abzugeben; und
die Befestigungseinrichtung eine Umhüllung (12) an dem distalen Ende des Abgabeelementes aufweist, wobei die Umhüllung ein offenes distales Ende besitzt, das in sich den Schaft des Anschlages aufnimmt.

2. Vorrichtung gemäß Anspruch 1, wobei das langgestreckte Abgabeelement eine Längsbohrung aufweist, die sich von seinem proximalen Ende zu seinem distalen Ende durch dieses hindurch erstreckt.

3. Vorrichtung gemäß Anspruch 2 und umfassend einen Handgriffbereich (16), der an dem proximalen Ende des langgestreckten Abgabeelementes angeordnet ist, um die Handhabung des Abgabesystems zu erleichtern, eine Längsbohrung, die sich durch den Handgriffbereich in axialer Ausrichtung mit der Längsbohrung des langgestreckten Abgabesystems erstreckt; und weiter umfassend eine Stangenelement, das verschiebbar in der Längsbohrung des langgestreckten Abgabeelementes und des Handgriffbereichs aufgenommen wird und dazu geeignet ist, sich in Richtung des distalen Endes des langgestreckten Abgabeelementes zu bewegen, wobei das Stangenelement einen proximalen Endbereich besitzt, der sich über ein proximales Ende des Handgriffbereichs hinaus erstreckt; und
wobei eine auf dem proximalen Endbereich des Stangenelement aufgebrachte distale Kraft dazu führt, daß das Stangenelement in distaler Richtung so versetzt wird, daß ein distales Ende des Stangenelementes in Eingriff tritt mit dem Anschlagschaft, um das Lösen der Anschlagkomponente aus ihrem Eingriff mit dem distalen Ende des langgestreckten Abgabeelementes zu bewirken.

4. Vorrichtung gemäß Anspruch 3, wobei eine äußere Umfangsoberfläche des Anschlagschafts in kraftschlüssigem Eingriff steht mit einer inneren Umfangswand des langgestreckten Abgabeelementes, um das Halten der Anschlagkomponente innerhalb des langgestreckten Abgabeelementes zu unterstützen.

5. Vorrichtung gemäß einem dem vorhergehenden Ansprüche, wobei ein Endstück an dem Anschlagschaft befestigt ist.

6. Vorrichtung gemäß einem der vorhergehenden Ansprüche wobei:
(a) der Anschlag ein Anschlagaufbau ist, der zur Verwendung mit einer Vorrichtung zum Durchführen einer kreisförmigen Anastomose geeignet ist, wobei der Anschlagschaft eine Anschlagstange mit proximalen und distalen Endbereichen umfaßt, der Anschlagkopf lösbar an dem distalen Enbereich der Anschlagstange angebracht ist, der distale Endbereich von einer ersten Betriebsposition zu einer zweiten nichtbetriebsfähigen Position schwenkbar ist, wobei zumindest eine Abmessung des Anschlagaufbaus in der zweiten nichtbetriebsfähigen Position wirksam geringer als die entsprechende Abmessung in der ersten Betriebsposition ist.

7. Vorrichtung gemäß einem der vorhergehenden Ansprüche, wobei der Anschlagschaft umfaßt eine Mehrzahl von sich in Längsrichtung erstreckenden äußeren Keilen (34'), die zwischen seinen proximalen und distalen Endbereichen angeordnet sind, wobei die äußeren Keile in Eingriff gebracht werden können mit zusammenwirkenden, sich in Längsrichtung erstreckenden, inneren Keilnuten, die innerhalb der Umhüllung gebildet sind, um die Anschlagstange mit der Vorrichtung ordnungsgemäß auszurichten.

8. Vorrichtung gemäß einem der vorhergehenden Ansprüche, wobei das distale Ende des Anschlagschafts eine Umfangsbefestigungshülse (40') umfaßt, wobei die Befestigungshülse innerhalb einer kreisförmigen Öffnung aufgenommen ist, die innerhalb des Anschlagkopfes gebildet ist, um den Anschlagkopf an dem Anschlagschaft anzubringen.

9. Vorrichtung gemäß Anspruch 8, wobei die Befestigungshülse eine Mehrzahl von sich in Längsrichtung erstreckenden äußeren Keilen (42') umfaßt, die in Eingriff zu bringen sind mit zusammenwirkenden, sich in Längsrichtung erstreckenden inneren Keilnuten, die innerhalb des Anschlagkopfes gebildet sind, um den Anschlagkopf mit dem Anschlagschaft ordnungsgemäß auszurichten.

10. Vorrichtung gemäß einem der vorhergehenden Ansprüche, wobei der Anschlagkopf lösbar am distalen Endbereich des Anschlagschafts befestigt ist, der distale Endbereich von einer ersten Betriebsposition in allgemeiner Ausrichtung mit einer Längsachse, die durch den Anschlagschaft proximal seines distalen Bereiches definiert ist, zu einer zweiten, nicht-betriebsfähigen Position schwenkbar ist, die winklig relativ zur Längsachse versetzt ist, wobei die Anordnung des Anschlagkopfes und Schafts eine wirksame Querschnittsfläche im allgemeinen quer zur Längsachse definiert und wobei die wirksame Querschnittsfläche des Anschlagaufbaus in der zweiten, nicht-betriebsfähigen Position geringer als die wirksame Querschnittsfläche des Anschlagaufbaus in der ersten Betriebsposition ist, um das Vorrücken des Anschlagaufbaus durch Körpergewebe zu erleichtern.

11. Vorrichtung gemäß Anspruch 10, wobei der distale Endbereich in Bezug auf die durch den Anschlagkopf definierte Längsachse über einen Winkel von bis zu etwa 90° schwenkbar ist.

12. Vorrichtung gemäß Anspruch 11, wobei der distale Endbereich dazu geeignet ist, sich bis zu etwa 90° in Bezug auf jede Seite der Längsachse zu verschwenken, wobei eine vollständige schwenkende Ablenkung derselben von etwa 180° vorgesehen ist.

13. Vorrichtung gemäß einem der vorhergehenden Ansprüche, wobei der proximale Endbereich des Anschlagschafts einen konisch geformten Befestigungsbereich (30') umfaßt, der gestaltet und dimensioniert ist, um das Anbringen des Anschlagschaftes an einer Aufnahmeröhre zu erleichtern.

14. Vorrichtung gemäß einem der vorhergehenden Ansprüche, wobei die Löseeinrichtung umfaßt ein Stangenelement, das innerhalb der Längsbohrung des langgestreckten Abgabeelementes aufgenommen ist und eine Anlageoberfläche (15) an ihrem distalen Ende besitzt, wobei das Stangenelement für eine distale Versetzung relativ zu dem langgestreckten Abgabeelement geeignet ist, so daß die Anlageoberfläche in Eingriff tritt mit der Anschlagkomponente, um eine distale Versetzung derselben und das Lösen der Anschlagkomponente von der Befestigungseinrichtung zu bewirken.

15. Vorrichtung gemäß einem der vorhergehenden Ansprüche, wobei die Befestigungseinrichtung eine kraftschlüssige Verbindung zwischen einer äußeren Umfangsoberfläche eines Anschlagschafts und einer inneren Umfangswand des langgestreckten Abgabelementes umfaßt.

16. Vorrichtung gemäß einem der vorhergehenden Ansprüche, wobei das langgestreckte Abgabeelement biegsam ist.

17. Anastomoseeinrichtung (60) umfassend eine Vorrichtung gemäß einem der vorhergehenden Ansprüche, die Einrichtungen aufweist, um Befestiger abzuschießen, die zweiteilige, absorbierbare Befestiger sind.

## Revendications

1. Appareil chirurgical (10) pour transmettre une enclume à un site de chirurgie dans une section d'organe tubulaire qui comporte :
une enclume (18) avec une tête d'enclume annulaire (20) et un arbre d'enclume axial (22) qui s'étend le long d'un axe d'une face transversa le de formation d'agrafes de la tête d'enclume (20);
un élément de transmission oblong;
un moyen de montage pour le montage amovible de l'enclume à une extrémité distale dudit élément de transmission oblong; et
un moyen de relâchement (14) pour libérer l'enclume dudit moyen de montage,
et caractérisé en ce que
le moyen de montage est agencé pour transmettre l'enclume dans un mouvement distal, la face de formation d'agrafes de l'enclume étant orientée proximalement; et
le moyen de montage comporte une gaine (12) à l'extrémité distale dudit moyen de transmission, la gaine ayant une extrémité distale ouverte qui reçoit dans celle-ci l'arbre d'enclume.

2. Appareil selon la revendication 1, où ledit élément de transmission oblong inclut un perçage longitudinal s'étendant à travers celui-ci, de son extrémité proximale à son extrémité distale.

3. Appareil selon la revendication 2 et incluant une portion de poignée (16) disposée à ladite extrémité proximale dudit élément de transmission oblong pour faciliter la manipulation du système de transmission, un perçage longitudinal s'étendant à travers ladite portion de poignée, suivant un alignement axial avec ledit perçage longitudinal dudit élément de transmission oblong; et incluant en outre, un élément de tige reçu de manière coulissante dans ledit perçage longitudinal dudit élément de transmission oblong et de ladite portion de poignée et apte à se déplacer vers ladite extrémité distale dudit élément de transmission oblong, ledit élément de tige ayant une portion d'extrémité proximale s'étendant au-delà d'une extrémité proximale de ladite portion de poignée; et
où une force distale appliquée à ladite portion d'extrémité proximale dudit élément de tige amène ledit élément de tige à être déplacé distalement de telle sorte qu'une extrémité distale dudit élément de tige vient en prise avec ledit arbre d'enclume pour produire le relâchement dudit composant d'enclume de sa prise avec ladite extrémité distale dudit élément de transmission oblong.

4. Appareil selon la revendication 3, où une surface périphérique extérieure dudit arbre d'enclume est mise en prise par friction avec la paroi périphérique intérieure dudit élément de transmission oblong pour contribuer à retenir ledit composant d'enclume dans ledit élément de transmission oblong.

5. Appareil selon l'une des revendications précédentes, où une queue est fixée audit arbre d'enclume.

6. Appareil selon l'une des revendications précédentes, dans lequel:
(a) l'enclume est un ensemble d'enclume apte à être utilisé avec un appareil pour exécuter une anastomose circulaire, ledit arbre d'enclume comportant une tige d'enclume avec des portions d'extrémité proximale et distale, ladite tête d'enclume étant montée amoviblement sur ladite portion d'extrémité distale de ladite tige d'enclume, ladite portion d'extrémité distale étant apte à pivoter d'une première position opératoire à une deuxième position non opératoire par quoi au moins une dimension dudit ensemble d'enclume dans ladite deuxième position non opératoire est effectivement plus petite que la dimension correspondante dans ladite première position opératoire.

7. Appareil selon l'une des revendications précédentes, où ledit arbre d'enclume comporte plusieurs cannelures extérieures (34') s'étendant longitudinalement disposées entre ses portions d'extrémité proximale et distale, lesdites canne dures extérieures pouvant être mises en prise avec des cannelures intérieures coopérantes s'étendant longitudinalement formées dans ladite gaine afin d'aligner correctement ladite tige d'enclume avec l'appareil.

8. Appareil selon l'une des revendications précédentes, où l'extrémité distale de l'arbre d'enclume comporte un collier de montage circonférentiel (40'), ledit collier de montage étant reçu dans une ouverture circulaire ménagée dans ladite tête d'enclume pour monter ladite tête d'enclume sur ledit arbre d'enclume.

9. Appareil selon la revendication 8, où ledit collier de montage comporte une pluralité de cannelures extérieures (42') s'étendant longitudinalement, pouvant être mises en prise avec des cannelures intérieures coopérantes s'étendant longitudinalement, formées dans ladite tête d'enclume pour aligner correctement ladite tête d'enclume avec ledit arbre d'enclume.

10. Appareil selon l'une des revendications précédentes, où la tête d'enclume est montée amoviblement sur la portion d'extrémité distale dudit arbre d'enclume, ladite portion d'extrémité distale pouvant pivoter d'une première position opératoire généralement alignée avec un axe longitudinal défini par ledit arbre d'enclume proche de sa portion distale à une deuxième position non opératoire déplacée angulairement relativement audit axe longitudinal par quoi l'ensemble formé par la tête et l'arbre d'enclume définit une zone effective en section transversale généralement transversale audit axe longitudinal et par quoi la zone effective en section transversale dudit ensemble d'enclume dans ladite deuxième position non opératoire est plus petite que la zone effective en section transversale dudit ensemble d'enclume dans ladite première position opératoire, afin de faciliter l'avance dudit ensemble d'enclume à travers le tissu corporel.

11. Appareil selon la revendication 10, où ladite port ion d'extrémité distale peut pivoter par rapport audit axe longitudinal défini par ledit arbre d'enclume suivant un angle allant jusqu'à environ 90°.

12. Appareil selon la revendication 11, où ladite portion d'extrémité distale est apte à effectuer un mouvement de pivotement sur environ 90° vers chaque côté dudit axe longitudinal par quoi une articulation pivotante de celle-ci est réaliséé sur environ 180°.

13. Appareil selon l'une des revendications précédentes, où la portion d'extrémité proximale de l'arbre d'enclume comporte une portion de montage de forme conique (30') configurée et dimensionnée pour faciliter le montage dudit arbre d'enclume sur un tube de réception.

14. Appareil selon l'une des revendications précédentes, où ledit moyen de relâchement comporte un élément de tige reçu dans ledit perçage longitudinal dudit élément de transmission oblong et comportant une surface de palier (15) à son extrémité distale, ledit élément de tige étant conçu en vue d'un déplacement distal relativement audit élément de transmission oblong de telle sorte que ladite surface de palier est mise en prise avec le composant d'enclume pour effectuer un déplacement distal de celui-ci ainsi qu'un relâchement du composant d'enclume dudit moyen de montage.

15. Appareil selon l'une des revendications précédentes, où ledit moyen de montage comprend un ajustement à friction entre une surface périphérique extérieure d'un arbre d'enclume et une paroi périphérique intérieure dudit élément de transmission oblong.

16. Appareil selon l'une des revendications précédentes, où ledit élément de transmission oblong est flexible.

17. Dispositif d'anastomose (60) incluant l'appareil tel que revendiqué dans l'une des revendications précédentes incluant un moyen pour tirer des attaches qui sont des attaches résorbables en deux parties.
